# EUROPEAN PATENT APPLICATION

(11) **EP 4 023 213 A1**
(43) Date of publication of application: **06.07.2022**
(21) Application number: 21217819.8
(22) Date of filing: 27.12.2021
(51) Int. Cl.: A61K 9/14, A61K 9/16, A61K 9/20, A61K 9/28, A61K 31/513

(54) **PHARMACEUTICAL COMPOSITIONS COMPRISING ALOGLIPTIN**

(30) Priority: 29.12.2020 TR 202022144
(71) Applicant: SANOVEL ILAC SANAYI VE TICARET A.S., Istanbul 34460 (TR)
(72) Inventor: BASARAN, Salih Sermet, Istanbul (TR); KOKSAL UZUN, Selin, Istanbul (TR); TOK, Gülcin, Istanbul (TR); SÜNEL, Fatih, Istanbul (TR)
(74) Representative: Sevinç, Erkan

(57) **Abstract**

The present invention relates to solid pharmaceutical compositions comprising alogliptin or a pharmaceutically acceptable salt thereof and at least one pharmaceutically acceptable polymer. The composition is obtained using an efficient process.

## Description

### Field of the invention

The present invention relates to pharmaceutical compositions comprising alogliptin or a pharmaceutically acceptable salt thereof and at least one pharmaceutically acceptable excipient. The composition is obtained using an efficient process.

### Background of the invention

Diabetes mellitus is a group of disorders of carbohydrate metabolism in which the action of insulin is diminished or absent through altered secretion and/or the insulin activity is decreased. Two main types of diabetes are Type 1 and Type 2:
Type 1 diabetes occurs because the insulin-producing cells of the pancreas (beta cells) are damaged. With Type 1 diabetes, the pancreas makes little or no insulin, so sugar cannot get into the body's cells for use as energy. People with Type 1 diabetes must use insulin injections to control their blood glucose.
With Type 2 diabetes, the pancreas makes insulin, but the produced insulin either is not sufficient or does not work properly. This type of diabetes occurs mostly in people who are over the age of 40 years and overweight. Type 2 diabetes may sometimes be controlled with a combination of diet, weight management, and exercise. However, treatment also may include oral glucose-lowering medications or insulin injections.

Alogliptin, 2-[[6-[(3-aminopiperidin-1-yl)-3-methyl-2,4-dioxopyrimidin-1-yl]methyl]benzonitrile, is an orally administered antidiabetic drug, having the chemical structure shown by Formula I below.

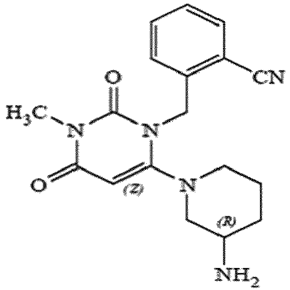

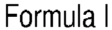

Alogliptin is disclosed in patent document nos. US 2005/261271, EP 1586571 and also WO 2005/095381. Patent document WO 2007/035629 discloses alogliptin in the form of the benzoate salt, hydrochloride salt, or tosylate salt. Polymorphs of alogliptin benzoate are disclosed in patent document WO 2007/035372. A process for preparing alogliptin is disclosed in patent document nos. WO 2007/112368 and WO 2007/035629 .

Stability, solubility and dissolution rate problems are seen in formulations in the prior art. In addition, there are problems encountered during the compression process in the production of tablets. Currently there is a need for developing an improved oral pharmaceutical composition of alogliptin which has content uniformity, high solubility, and thus high bioavailability and long-term stability.

Therefore, there is an outstanding need for a new pharmaceutical composition preferably comprising alogliptin.

### Detailed Description of the Invention

The main object of the present invention is to provide pharmaceutical compositions with high solubility, high bioavailability, high physical and chemical stability and a long shelf life, using an efficient process.

Another object of the present invention is to provide pharmaceutical compositions with high content uniformity.

The term "alogliptin" as used herein means alopgliptin in free base form or in the form of pharmaceutically acceptable salt, crystalline polymorph, solvate, hydrate or ester or in an amorphous form or in the form of mixtures thereof.

Alogliptin is poorly soluble in water (10 mg/ml at 25 °C) and exhibits relatively low oral bioavailability.

In the present invention, the pharmaceutical composition is suitable for oral administration. Solid oral dosage form is used for bioavailability. Suitable solid oral dosage forms are selected from the group consisting of tablets, granules, powders, pellets or unit dosage packs.

In the present invention, the pharmaceutical compositions comprise alogliptin and at least one pharmaceutically acceptable polymer, wherein the composition is obtained using a hot melt method. The said polymers are suitable for processing by hot melt extrusion.

Hot melt extrusion is a prominent technology in the pharmaceutical industry. The methods used in the state of the art can also be preferred in the invention. By reducing the process steps, hot melt extrusion offers a potential for shorter and more efficient time usage for the final product. Hot melt extrusion is used to disperse the active agent in a matrix at molecular level, thereby forming solid solutions. This method is used to obtain the desired dissolution rate and stability for the poorly soluble active agent.

Hot melt extrusion is a technique for producing amorphous solid dispersions in which the active agent is heated or dissolved in a dispersion carrier and stabilized by mixing.

According to one embodiment of the present invention, pharmaceutically acceptable polymer is one or more selected from vinyl pyrrolidone-vinyl acetate copolymer, dimethyl amino ethyl methacrylate copolymer, microcrystalline cellulose, polyvinyl caprolactam-polyvinyl acetate-polyethylene glycol graft copolymer, or mixtures thereof.

According to one embodiment of the present invention, said polymer is preferably vinyl pyrrolidone-vinyl acetate copolymer.

According to one embodiment of the present invention, another said polymer is preferably dimethyl amino ethyl methacrylate copolymer.

According to one embodiment of the present invention, another said polymer is preferably microcrystalline cellulose.

According to one embodiment of the present invention, another said polymer is preferably polyvinyl caprolactam-polyvinyl acetate-polyethylene glycol graft copolymer.

According to one embodiment of the present invention, the weight ratio of alogliptin to pharmaceutically acceptable polymer is between 0.7 and 1.3, preferably between 0.8 and 1.2, more preferably between 0.9 and 1.1. The diluent microcrystalline cellulose mentioned in Example 3 is calculated here by ignoring the ranges of ratio alogliptin/pharmaceutical polymer mentioned herein.

According to one embodiment of the present invention, the composition further comprises at least one pharmaceutically acceptable excipient selected from diluents, dispersants, lubricants, glidants, plasticizers or mixtures thereof.

According to one embodiment of the present invention, the diluent is selected from the group comprising microcrystalline cellulose, mannitol, lactose, starch, calcium phosphate dihydrate, calcium phosphate trihydrate, dibasic calcium phosphate, calcium carbonate, calcium sulfate, carboxymethyl cellulose calcium, powdered cellulose, cellulose acetate, pregelatinized starch, lactose monohydrate, corn starch, or mixtures thereof.

According to one embodiment of the present invention, said formulation comprises at least one coating layer.

According to one embodiment of the present invention, said coating layer comprises hypromellose and/or polyethylene glycol. A suitable amount of a mixture of both is preferred in the invention.

The method described above is used both to provide a uniform formulation content and to eliminate the need for any liquid solvents, including water. As such all fluidity problems encountered during production are also prevented. In said formulation, the weight ratio of alogliptin to pharmaceutically acceptable polymer is in the ranges which allow the desired dissolution rate and solubility to be achieved. Polymers with low glass transition temperature and melting point are used in the said formulation.

Suitable coating materials are selected from the group comprising hydroxypropylmethyl cellulose (hypromellose), hydroxypropyl cellulose, polyvinyl alcohol (PVA), polyethylene glycol (PEG), talc, polyvinyl alcohol-polyethylene glycol copolymers (Kollicoat IR), polyvinylpyrrolidone, polyvinylpyrrolidone-vinyl acetate copolymer (PVP-VA) and all variants of OpadryTM, pigments, dyes, copolymers of titanium dioxide, iron oxide or polymethylmethacrylate (Eudragit) or mixtures thereof.

In a preferred embodiment, plasticizer is selected from among polyoxyethylene sorbitan fatty acid esters, which are polysorbate 20, polysorbate 21, polysorbate 40, polysorbate 60, polysorbate 61, polysorbate 65, polysorbate 80, polysorbate 81, polysorbate 85, polysorbate 120, triacetin, citrate ester and low molecular weight polyethylene glycols or mixtures thereof.

In a preferred embodiment, plasticizer is polysorbate 80 (Tween 80).

In a preferred embodiment, plasticizer is triacetin.

In one embodiment, temperature can be lowered using a plasticizer during the hot melt extrusion method.

The use of plasticizers reduces the glass transition temperature and increases the stability of the active agent. The plasticizer used in the hot melt method lowers the glass transition temperature of the polymers used in hot melt method and thus allows formulation of the active agent at low temperatures. As a result, the formulation is formed more stable.

Preferably, the heat treatment is limited to a temperature equal to the melting temperature of the mixture to ensure the homogeneity of the composition before the melt product is cooled down to obtain the solid product.

The temperature is chosen according to the properties of the polymers and alogliptin.

**Table 1: Temperature zones during hot melt extrusion**

| **Zone 1** | **Zone 2** | **Zone 3** | **Zone 4** | **Zone 5** | **Zone 6** | **Zone 7** | **Zone 8** |
|---|---|---|---|---|---|---|---|
| 20-80 °C | 60-120 °C | 100-180 °C | 120-200 °C | 140-220 °C | 150-230 °C | 155-235 °C | 160-240 °C |

The aforesaid temperature ranges are suitable to provide high solubility, high physical and chemical stability in the present invention.

The composition according to this preferred embodiment comprises;
a. 1.00-10.00% by weight of alogliptin
b. 1.00-15.00% by weight of vinyl pyrrolidone-vinyl acetate copolymer
c. 1.00-10.00% by weight of croscarmellose sodium
d. 30.00-70.00% by weight of microcrystalline cellulose
e. 20.00-40% by weight of mannitol
f. 0.10-5.00% by weight of magnesium stearate
g. 1.00-5.00% by weight of coating material.

The composition according to this preferred embodiment comprises;
a. 1.00-10.00% by weight of alogliptin
b. 1.00-15.00% by weight of dimethyl amino ethyl methacrylate copolymer
c. 1.00-10.00% by weight of croscarmellose sodium
d. 30.00-70.00% by weight of microcrystalline cellulose
e. 20.00-40% by weight of mannitol
f. 0.10-5.00% by weight of magnesium stearate
g. 1.00-5.00% by weight of coating material.

The composition according to this preferred embodiment comprises;
a. 1.00-10.00% by weight of alogliptin
b. 1.00-15.00% by weight of microcrystalline cellulose
c. 1.00-10.00% by weight of croscarmellose sodium
d. 30.00-70.00% by weight of microcrystalline cellulose
e. 20.00-40% by weight of mannitol
f. 0.10-5.00% by weight of magnesium stearate
g. 1.00-5.00% by weight of coating material.

The composition according to this preferred embodiment comprises;
a. 1.00-10.00% by weight of alogliptin
b. 1.00-15.00% by weight of polyvinyl caprolactam-polyvinyl acetate-polyethylene glycol graft copolymer
c. 1.00-10.00% by weight of croscarmellose sodium
d. 30.00-70.00% by weight of microcrystalline cellulose
e. 20.00-40% by weight of mannitol
f. 0.10-5.00% by weight of magnesium stearate
g. 1.00-5.00% by weight of coating material.

Feed rate and screw speed values are important to provide homogeneity in the extrudate.

However, the screw speed value minimizes the risk of thermal degradation of alogliptin. The following values give the best stability result for the composition.

The hot melt extrusion temperature is in the range of about 40°C to about 250°C, preferably 100°C to 180°C.

In the present invention, the rotation speed of the screw is between 100 rpm and 150 rpm, 150 rpm and 200 rpm, 200 rpm and 250 rpm, 250 rpm and 300 rpm, 300 rpm and 350 rpm.

In this invention, the feed rate during the hot melt extrusion process is between 10 g/min and 15 g/min, 15 g/min and 20 g/min, 20 g/min and 25 g/min, 25 g/min and 30 g/min, 30 g/min and 35 g/min.

In the present invention, the torque value during the hot melt extrusion process is between 20% and 30%.

The compositions of the invention may be developed in an oral dosage form with immediate release, extended release, sustained release, controlled release, modified release and delayed release, or a combination thereof. Such compositions may be prepared using rate controlling polymers.

### Example 1: tablet

| **Ingredients** | **(%) Amount (w/w)** |
|---|---|
| Alogliptin | 1- 10 |
| Vinyl pyrrolidone-vinyl acetate copolymer, | 1-15 |
| Croscarmellose sodium | 1.00-10.00 |
| Mannitol | 20.00-40.00 |
| Microcrystalline cellulose | 30.00-70.00 |
| Magnesium stearate | 0.10-5.00 |
| **Total** | **100** |
| Coating | 1.00-5.00 |
| **Total coated tablet** | **101.00-105.00** |

| | |
|---|---|
| Kollidon va64 : Vinyl pyrrolidone-vinyl acetate copolymer | |

### Example 2: tablet

| **Ingredients** | **(%) Amount (w/w)** |
|---|---|
| Alogliptin | 1- 10 |
| Dimethyl amino ethyl methacrylate copolymer | 1-15 |
| Croscarmellose sodium | 1.00-10.00 |
| Mannitol | 20.00-40.00 |
| Microcrystalline cellulose | 30.00-70.00 |
| Magnesium stearate | 0.10-5.00 |
| **Total** | **100** |
| Coating | 1.00-5.00 |
| **Total coated tablet** | 101.00-105.00 |

| | |
|---|---|
| Eudrogit EPO : Dimethyl amino ethyl methacrylate copolymer | |

### Example 3: tablet

| **Ingredients** | **(%) Amount (w/w)** |
|---|---|
| Alogliptin | 1- 10 |
| Microcrystalline cellulose (PH101) | 1-15 |
| Croscarmellose sodium | 1.00-10.00 |
| Mannitol | 20.00-40.00 |
| Microcrystalline cellulose | 30.00-70.00 |
| Magnesium stearate | 0.10-5.00 |
| **Total** | **100** |
| Coating | 1.00-5.00 |
| **Total coated tablet** | 101.00-105.00 |

### Example 4: tablet

| **Ingredients** | **(%) Amount (w/w)** |
|---|---|
| Alogliptin | 1- 10 |
| polyvinyl caprolactam-polyvinyl acetate-polyethylene glycol graft copolymer | 1-15 |
| Croscarmellose sodium | 1.00-10.00 |
| Mannitol | 20.00-40.00 |
| Microcrystalline cellulose | 30.00-70.00 |
| Magnesium stearate | 0.10-5.00 |
| **Total** | **100** |
| Coating | 1.00-5.00 |
| **Total coated tablet** | 101.00-105.00 |

| | |
|---|---|
| Soluplus: polyvinyl caprolactam, polyvinyl acetate, polyethylene glycol graft copolymers | |

The pharmaceutical formulations of the invention are prepared using the following steps in detail:

### Preparation of the extrudate

a. Mixing Alogliptin and Soluplus,
b. Melting this mixture in a hot melt extruder so that the alogliptin is dispersed in molecular level in the polymer to form the extrudate.

### Preparation of tablets

a. Powdering and sieving the extrudate,
b. Mixing extrudate powder, croscarmellose sodium, mannitol and microcrystalline cellulose,
c. Adding magnesium stearate to the mixture and then mixing,
d. Compressing the mixture into tablets
e. Coating these tablets

### Example 5: tablet

| **Ingredients** | **(%) Amount (w/w)** |
|---|---|
| Alogliptin | 1- 10 |
| One or a mixture selected from among vinyl pyrrolidone-vinyl acetate copolymer, dimethyl amino ethyl methacrylate copolymer; Microcrystalline cellulose; Polyvinyl caprolactam-polyvinyl acetate-polyethylene glycol graft copolymer | 1-15 |
| Plasticizer, preferably Polyoxyethylene sorbitan monooleate or triacetin | 1 - 2 |
| Croscarmellose sodium | 1.00-10.00 |
| Mannitol | 20.00-40.00 |
| Microcrystalline cellulose | 30.00-70.00 |
| Magnesium stearate | 0.10-5.00 |
| **Total** | **100** |
| Coating | 1.00-5.00 |
| **Total coated tablet** | 101.00-105.00 |

| | |
|---|---|
| Tween 80 : Polyoxyethylene sorbitan monooleate | |

The pharmaceutical formulations of the invention are prepared using the following steps in detail:

### Preparation of the extrudate

c. Mixing Alogliptin, Soluplus and Tween 80,
d. Melting this mixture in a hot melt extruder so that the alogliptin is dispersed in molecular level in the polymer to form the extrudate.

### Preparation of tablets

f. Powdering and sieving the extrudate,
g. Mixing extrudate powder, croscarmellose sodium, mannitol and microcrystalline cellulose,
h. Adding magnesium stearate to the mixture and then mixing
i. Compressing the mixture into tablets
j. Coating these tablets

Said coating material mainly consists of hypromellose and/or polyethylene glycol, the stability of the formulation is increased by the use of these excipients individually or in a mixture in the coating. The sample coating formulation is as follows;

| **Coating Material** |
|---|
| Hypromellose |
| Macrogol 8000 |
| Titanium dioxide (E171) |
| Yellow iron oxide (E172) |

The coating process is carried out using the processes already known in the art.

The formulation is administered in a dosage form of tablets of 6.25 mg, 12.5 mg and 25 mg.

## Claims

1. A hot melt extrusion formulation, **characterized in that** it comprises alogliptin or pharmaceutically acceptable salts, polymorphs, solvates, hydrates or esters thereof and at least one pharmaceutically acceptable polymer.

2. The pharmaceutical composition according to claim 1, wherein pharmaceutically acceptable polymer is one or more selected from among vinyl pyrrolidone-vinyl acetate copolymer, dimethyl amino ethyl methacrylate copolymer, microcrystalline cellulose, polyvinyl caprolactam-polyvinyl acetate-polyethylene glycol graft copolymer, or comprises mixtures thereof.

3. The pharmaceutical composition according to claim 2, wherein the said polymer is preferably vinyl pyrrolidone-vinyl acetate copolymer.

4. The pharmaceutical composition according to claim 2, wherein another said polymer is preferably dimethyl amino ethyl methacrylate copolymer.

5. The pharmaceutical composition according to claim 2, wherein another said polymer is preferably microcrystalline cellulose.

6. The pharmaceutical composition according to claim 2, wherein another said polymer is preferably polyvinyl caprolactam-polyvinyl acetate-polyethylene glycol graft copolymer.

7. The pharmaceutical composition according to claim 1, wherein the weight ratio of alogliptin to pharmaceutically acceptable polymer is between 0.7 and 1.3, preferably between 0.8 and 1.2, more preferably between 0.9 and 1.1.

8. The pharmaceutical composition according to claim 1, wherein the composition further comprises at least one pharmaceutically acceptable excipient selected from among diluents, dispersants, lubricants, glidants, plasticizers or mixtures thereof.

9. The pharmaceutical composition according to claim 1, wherein the said formulation further comprises at least one coating layer.

10. The pharmaceutical composition according to claim 9, wherein the said coating layer comprises hypromellose and/or polyethylene glycol.

11. Pharmaceutical composition according to any one of the preceding claims, wherein the composition comprises;
h. 1-10.00% by weight of alogliptin
i. 1-15.00% by weight of one or more selected from among vinyl pyrrolidone-vinyl acetate copolymer, dimethyl amino ethyl methacrylate copolymer, microcrystalline cellulose, polyvinyl caprolactam-polyvinyl acetate-polyethylene glycol graft copolymer, or mixtures thereof
j. 1.00-10.00% by weight of croscarmellose sodium
k. 30.00-70.00% by weight of microcrystalline cellulose
I. 20.00-40% by weight of mannitol
m. 0.10-5.00% by weight of magnesium stearate
n. 1.00-5.00% by weight of coating material

12. A hot melt extrusion process for preparing the pharmaceutical composition according to any one of the preceding claims, further comprises;
o. Mixing alogliptin and one selected from among vinyl pyrrolidone-vinyl acetate copolymer, dimethyl amino ethyl methacrylate copolymer, microcrystalline cellulose, polyvinyl caprolactam-polyvinyl acetate-polyethylene glycol graft copolymer
a. Melting this mixture in a hot melt extruder so that the alogliptin is dispersed in molecular level in the polymer to form the extrudate
b. Powdering and sieving the extrudate
c. Mixing extrudate powder, croscarmellose sodium, mannitol and microcrystalline cellulose
d. Adding magnesium stearate to the mixture and then mixing
e. Compressing the mixture into tablets
f. Coating these tablets
